# EUROPEAN PATENT APPLICATION

(11) **EP 4 578 495 A1**
(43) Date of publication of application: **02.07.2025**
(21) Application number: 23461700.9
(22) Date of filing: 27.12.2023
(51) Int. Cl.: A61N 5/10, A61B 90/30, A61B 90/00

(54) **A METHOD AND SYSTEM FOR POSITIONING A PATIENT**

(71) Applicant: MedApp Radiotherapy Sp. z o.o., 30-150 Krakow (PL)
(72) Inventor: Skalski, Andrzej, 32-091 Kozierow (PL); Stanuch, Maciej, 31-356 Krakow (PL)
(74) Representative: Kancelaria Eupatent.pl Sp. z.o.o

(57) **Abstract**

A method for positioning a patient (11) on a table (10) in preparation for radiation therapy to be delivered by a radiotherapy apparatus, the method comprising: registering (101), at a head-mounted display, HMD (15) worn by a medical practitioner (14), a position of a reference point (31) of a radiotherapy apparatus; providing (102) diagnostic imaging data (23) of the patient, including the patient's diagnostic position, wherein the diagnostic imaging data (23) is defined with reference to a diagnostic reference point (22); generating (103), by the visualization engine, a holographic visualization of the patient's diagnostic imaging position based on the diagnostic imaging data; and displaying (104) the holographic visualization of the patient's diagnostic imaging position (13) over the patient (11) using augmented reality or mixed reality technology of the HMD (15) to allow the medical practitioner (14) to align the current position of the patient with the patient's diagnostic imaging position (13).

## Description

### TECHNICAL FIELD

The present invention relates to the technical field of patient treatment, specifically focusing on the use of radioactive therapy, in particular radiation therapy. It is generally based on pre-treatment plans derived from patient imaging studies, which may include computed tomography (CT), magnetic resonance imaging (MRI), rotational angiography, cone-beam computed tomography (CBCT), Positron emission tomography (PET) and radiotherapy plan.

### BACKGROUND

Within the modern medical landscape, the use of radioactive beams for patient treatment has become a standard procedure. This process necessitates the formulation of a comprehensive treatment plan, pinpointing the precise location where the beam will interact with the patient's internal organ, in particular cancer tissue. This location is ascertained based on patient-specific models, constructed using one or more selected imaging techniques.

Computed Tomography (CT) employs a series of X-ray images taken from various angles around the body, coupled with computer processing, to generate cross-sectional images of bones, blood vessels, and soft tissues. Magnetic Resonance Imaging (MRI) leverages a magnetic field and radio waves to produce detailed images of the organs and tissues within the body. Rotational Angiography employs a series of X-ray images taken from different angles around the body to generate a detailed, three-dimensional image of the blood vessels. Cone-Beam Computed Tomography (CBCT) is a variant of traditional computed tomography that uses a cone-shaped X-ray beam to create a three-dimensional image of the patient's anatomy. Positron emission tomography (PET) constitutes an imaging modality designed to assess physiological function by detecting the radioactivity emitted following the introduction of a minute quantity of a radioactive tracer.

The use of these imaging techniques has been instrumental in the field of radiation therapy, as they facilitate the precise targeting of the radiation beam. This precision ensures that the therapeutic effect is maximized while minimizing the damage to the surrounding healthy tissues.

However, a significant challenge emerges due to the potential discrepancy between the patient's position during the diagnostic imaging and the actual radiation therapy. This discrepancy can lead to inaccuracies in the delivery of the radiation, which can compromise the treatment's effectiveness and increase the risk of damage to healthy tissues.

To illustrate this issue, consider a scenario of diagnostic imaging in female patients diagnosed with breast cancer. During the imaging process, the patient's arm is elevated to better expose the breast tissues. This position allows for a detailed and comprehensive imaging of the breast, which subsequently facilitates the formulation of an accurate treatment plan. However, when the patient arrives for therapy and lies on the table with her arms at her sides, her tissues may be arranged differently than during the diagnostic imaging when her arm was elevated.

This shift in position can significantly alter the configuration of the breast tissues, leading to a mismatch between the treatment plan based on the diagnostic images and the actual position of the tissues during treatment. This mismatch can result in the radiation beam not accurately targeting the intended tissues, diminishing the effectiveness of the treatment and potentially inflicting unnecessary damage to healthy tissues.

Moreover, this problem is not exclusive to breast cancer treatment but can arise in any situation where the patient's position during imaging differs from the position during treatment, such as during radiotherapy treatment of lung cancer, liver cancer, kidney cancer, brain tumors or neck cancer and other. This discrepancy can be attributed to various factors, such as patient discomfort in the imaging position, involuntary movements, or physiological changes between the imaging and treatment sessions.

In summary, while the employment of imaging techniques such as those mentioned above has significantly enhanced the accuracy and efficacy of radiation therapy, the issue of position discrepancy between the imaging and treatment sessions remains a substantial challenge. This problem can lead to inaccuracies in the radiation delivery, compromising the effectiveness of the treatment and increasing the risk of damage to healthy tissues.

### SUMMARY OF THE INVENTION

There is a need for a solution that ensures accurate alignment between the patient's position during diagnostic imaging (also referred to as pretreatment imaging or preoperative imaging) and during radiation therapy. Such a solution would significantly improve the accuracy and effectiveness of radiation therapy, enhancing patient outcomes and reducing the risk of damage to healthy tissues. This invention aims to address this need, providing a novel approach to patient positioning in radiation therapy that ensures accurate delivery of the radiation beam.

The main concept of the present invention, in contrast to prior art solutions which were directed to aligning the radiotherapy plan to the patient's position on the table (wherein the position is meant to refer both to patient's location and patient's pose), is to align the patient so that the patient's position corresponds to the position at which the diagnostic imaging data were collected and on which the radiotherapy plan is based.

The object of the invention is a method for positioning a patient on a table in preparation for radiation therapy to be delivered by a radiotherapy apparatus. This method includes registering a position of a reference point of the radiotherapy apparatus at a head-mounted display (HMD) worn by a medical practitioner, providing diagnostic imaging data of the patient, generating a holographic visualization of the patient's diagnostic imaging position, and displaying the holographic visualization over the patient using augmented reality or mixed reality technology of the HMD to align the patient's position with the diagnostic imaging position.

A preferred embodiment of the invention includes providing, along with the diagnostic imaging data, a radiotherapy plan that includes the location of the planning target volume and displaying this volume via the HMD. This embodiment offers the technical advantage of allowing the medical practitioner to ascertain that the patient is correctly positioned with respect to the planning target volume.

Another preferred embodiment involves system-assisted indicating, during the alignment of the patient's position, a level of compliance between the patient's current position and the holographic visualization of the diagnostic position. The technical advantage here is the provision of real-time feedback to the medical practitioner, enabling immediate and accurate adjustments to the patient's position.

A further preferred embodiment includes generating a secondary image of the patient's diagnostic imaging position to be displayed outside the radiotherapy room. The technical advantage of this feature is that it allows other medical staff to monitor and verify the patient's positioning without being present in the radiotherapy room, facilitating a collaborative treatment environment.

The invention also relates to a system for positioning a patient. The system comprises an HMD to be worn by a medical practitioner and a computer workstation with a planning tool and a visualization engine configured to perform the steps of the method described herein.

A preferred aspect of the invention is the use of a reference point indicator designed to establish a reference point corresponding to the gantry reference point of the radiotherapy apparatus. The technical advantage of this aspect is that it ensures precise alignment of the diagnostic imaging data with the coordinate system of the radiotherapy apparatus.

Another preferred aspect includes laser pointers for emitting laser rays that intersect at the gantry reference point, providing a technical advantage by enabling the accurate and easy identification of the gantry reference point for alignment purposes.

Embodiments of the invention may also comprise a reference point position recognition module configured to register the position of the head of the reference point indicator, offering the technical advantage of automating the process of aligning the holographic visualization with the patient's position.

The system may comprise a wireless communication interface for transmitting holographic data from the workstation to the HMD, providing the technical advantage of enhancing the mobility of the medical practitioner during the patient positioning process.

A further preferred aspect relates to a position evaluation module configured to determine compliance between the patient's current position and the holographic visualization, providing feedback on the level of compliance. This aspect offers the technical advantage of facilitating accurate patient positioning by providing immediate and actionable feedback to the medical practitioner.

In some embodiments, the feedback provided by the position evaluation module includes audio signals or visual modifications of the holographic image, which presents the technical advantage of intuitive and easily interpretable guidance for the medical practitioner during patient positioning.

The system may further include a 3D visualization module located outside the radiotherapy room for displaying a secondary image, offering the technical advantage of enabling additional visualization options for medical staff and facilitating communication and coordination during the patient positioning process.

These and other features, aspects, and advantages of the invention will become better understood with reference to the following drawings, descriptions, and claims.

### BRIEF DESCRIPTION OF DRAWINGS

The invention is shown by means of example embodiments in a drawing, wherein:
Figs. 1A and 1B show schematically a first example wherein a patient is positioned in an incorrect and correct position, respectively;
Figs. 2A and 2B show a second example as a view from an HMD wherein a body fragment is positioned in an incorrect and correct position, respectively;
Fig. 3 shows a schematic of a system for positioning a patient;
Fig. 4 shows an example of a reference point indicator;
Figs. 5A and 5B illustrate registering a reference point and diagnostic imaging data visualized in accordance with the registered reference point;
Fig. 6 shows steps of a method for positioning the patient.

### DETAILED DESCRIPTION

The following detailed description is of the best currently contemplated modes of carrying out the invention. The description is not to be taken in a limiting sense but is made merely for the purpose of illustrating the general principles of the invention.

The primary inventive concept of this invention is to devise a system and method that facilitates the alignment of a patient, prior to radiation therapy, to the position assumed during the imaging process which formed the basis for the radiotherapy plan. Such alignment enhances the congruence between the patient's anatomy during the radiation therapy, particularly the soft tissues, and the radiotherapy plan.

The radiotherapy plan defines volumes that should be subject to radiation, such as the gross tumor volume (GTV) and a margin for sub-clinical disease spread which therefore cannot be fully imaged - this is known as the clinical target volume (CTV). The CTV must be adequately treated to achieve a cure. Furthermore, the radiotherapy plan defines a third volume - the planning target volume (PTV), which allows for uncertainties in planning or treatment delivery. It is a geometric concept designed to ensure that the radiotherapy dose is actually delivered to the CTV In addition, the radiotherapy plan may include volumes of organs at risk (OAR), i.e., structures that should be protected from radiation. Therefore, in summary, the radiotherapy plan defines the volumetric distribution of radiation dose that should be concentrated on the PTV and should avoid the OAR volumes.

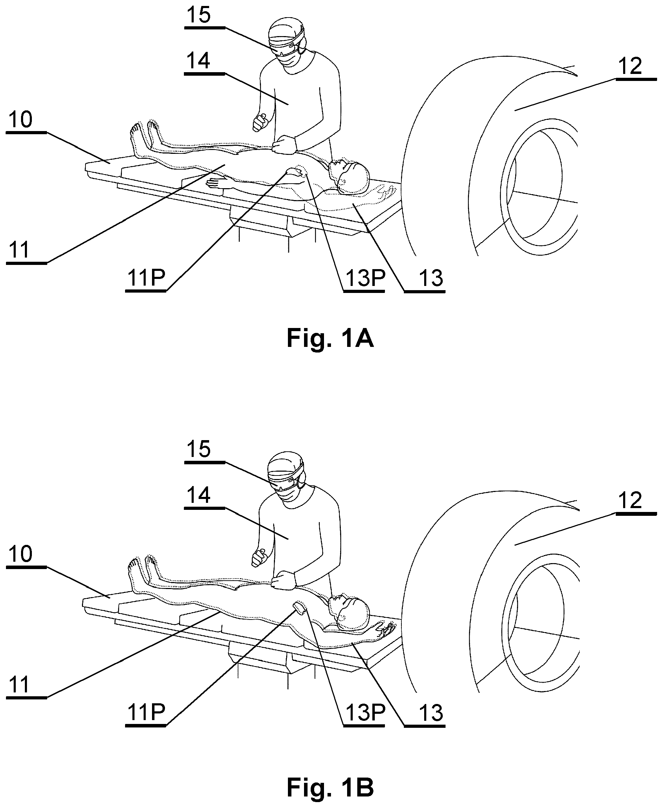

This concept is exemplified in Figures 1A and 1B. These figures depict a patient 11, positioned on a table 10, in proximity to a gantry 12. In Fig. 1A, the patient's current position (marked in solid line), including the PTV 11P to be radiated, deviates from the diagnostic imaging position 13 (marked in dotted line) assumed during diagnostic imaging, including the PTV 13P as defined by the radiotherapy plan based on the diagnostic imaging 13.

This diagnostic imaging position 13, along with the position of the PTV 13P, can be visualized by a medical practitioner 14 wearing a head-mounted display (HMD), such as augmented reality or mixed reality goggles 15. This visualization can be achieved using augmented reality (AR) or mixed reality (MR) technology, which overlays a three-dimensional semi-transparent outline and volumetric representation of the diagnostic imaging position 13 onto the real image of the patient's current position. In this example, it is evident that the patient's current position 11, with both hands along the body, significantly deviates from the diagnostic imaging position 13, where the patient had one arm raised. Consequently, the practitioner 14 can guide or set the patient to adjust their body parts to match the diagnostic imaging position 13, as shown in Fig. 1B, thereby increasing the alignment of the actual patient's PTV 11O with the diagnostic PTV position 13P. This alignment allows the gantry 12 to facilitate correct delivery of radiation by the gantry 12.

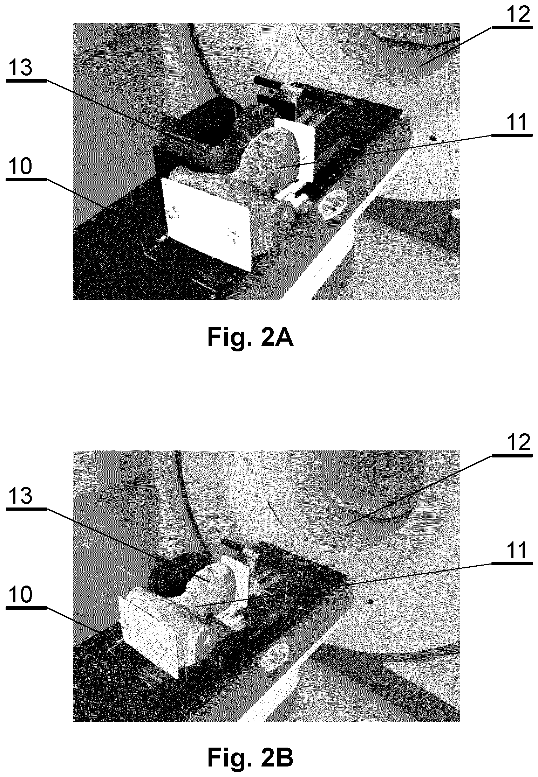

The concept is further exemplified in Figs. 2A and 2B. These figures present an image as viewed by the medical practitioner 14 through the HMD 15. Fig. 2A shows a patient's head 11 (for the sake of data protection issues, a head phantom is presented instead of a real person) arranged, positioned on the table 10. The current position of the patient's head 11 deviates from the diagnostic imaging position 13. The practitioner 14 can then guide the patient to adjust the position of their head 11, or the position of the table 10, to align the patient with the diagnostic imaging position 13, as shown in Fig. 2B.

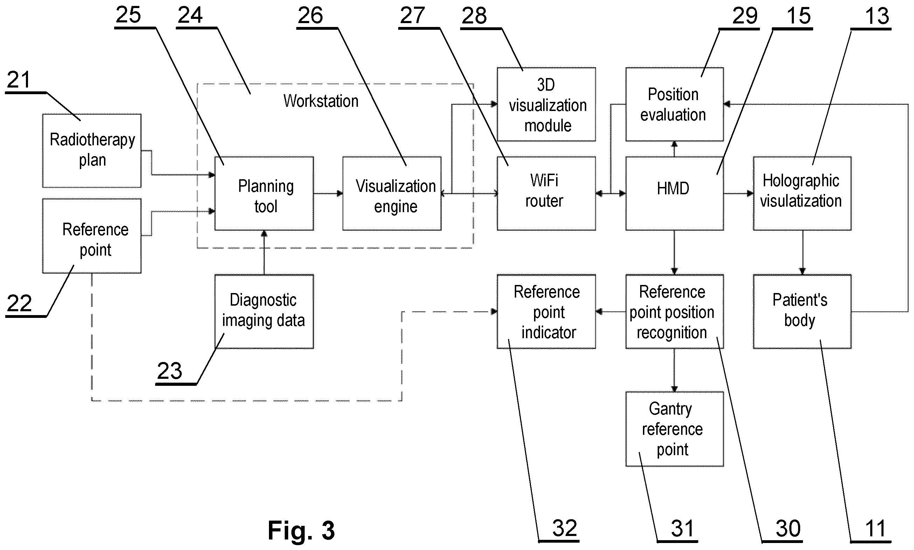

Fig. 3 shows a schematic of a system for positioning a patient. The system comprises a computer workstation 24, which can be a standard computer with a processor and memory storing data and software that facilitate the visualization or cloud solution. The computer workstation 24 operates a planning tool 25, such as a software application, which collects diagnostic imaging data 23 and allows a medical practitioner to define a radiotherapy plan 21 based on the diagnostic imaging data 23. The plan 21 can be delivered from other, external planning tool. The diagnostic imaging data 23 are spatially defined in accordance with the position of a reference point 22. The output of the planning tool 25, including data defining the patient's diagnostic position 13 and the diagnostic PTV position 13P is input to a visualization engine 26, such as a software application for 3D rendering of holographic data.

The holographic data is transmitted, preferably wirelessly by a wireless communication interface, such as a Wi-Fi router 27, to the HMD 15 worn by the medical practitioner 14 who prepares the patient for radiotherapy, and is displayed by the HMD as a holographic visualization including the diagnostic position 13 and the diagnostic PTV position 13P as shown in Figs. 1A, 1B, 2A, 2B over the patient's body 11. An additional 3D visualization module 28 can be provided to generate a secondary image, such as a display screen providing images outside the radiotherapy room or another HMD. Based on the actual position of the patient's body 11 and the diagnostic position 13, the position of the patient can be evaluated either by the medical practitioner 14 or by a dedicated position evaluation module 29, which can be auxiliary or integrated with the computer workstation 24.

The position evaluation module 29 may be configured to determine the compliance between the position of the patient 11 with the holographic visualization of the diagnostic position 13, indicate the level of compliance and/or make suggestions on how the patient should be repositioned in order to match the diagnostic position. The indication made by the position evaluation module 29 may take various forms, such as an audio signal (e.g., in a form of series of audio impulses, the more frequent the more compliant the position or in a form of modification of the holographic image displayed, for example highlighting the holographic image (in fragments or in total) to a green color if the actual position of the patient is compliant with the diagnostic position).

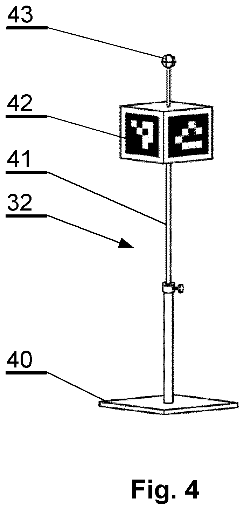

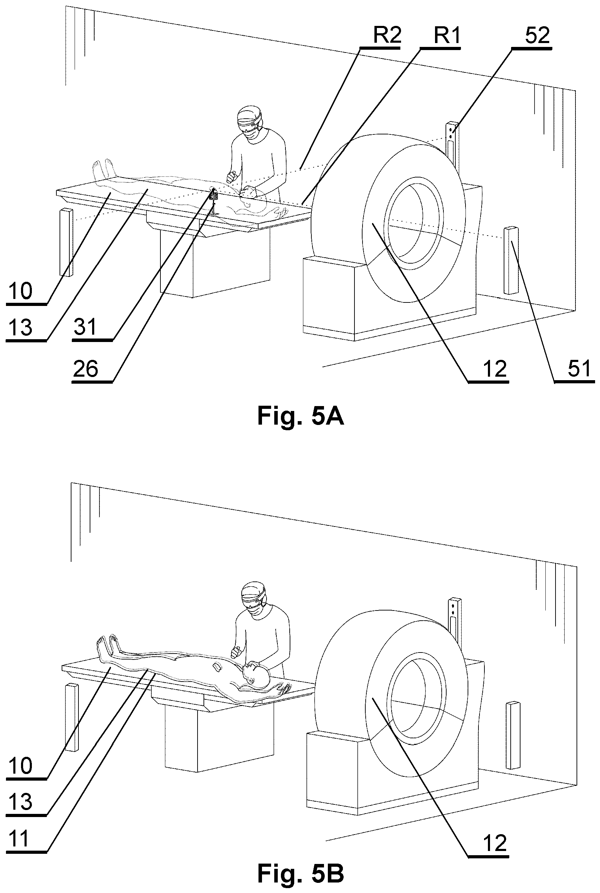

In order to precisely align the diagnostic image with the table 10 on which the radiotherapy will be performed, a reference point indicator 32, such as shown in Fig. 4, can be used to align the main reference point of the radiotherapy apparatus with the reference point 22 based on which the diagnostic imaging data 23 were generated. The reference point of the radiotherapy apparatus (also referred to as the gantry reference point) 31 is usually at an isocenter, i.e., a point of crossing of the gantry rotation axis of the therapeutic apparatus and the central axis. For example, the radiotherapy apparatus may comprise a system of laser pointers 51, 52 that emit laser rays R1, R2 that intersect at the reference point (isocenter) of the radiotherapy apparatus. Before the patient is placed on the table 10, the reference point indicator 32 should be positioned such that its head 43 is located at the reference point, as shown in Fig. 5A. Next, the HMD 15 is configured to register the position of the head 43 by means of a reference point position recognition module 31. Knowing the position of the head 43, and therefore the reference point of the radiotherapy apparatus, the holographic visualization of the diagnostic image 13 can be aligned by aligning the diagnostic image reference point 22 with the registered position of the head 43. The presence of the reference point indicator 32 is no longer necessary and it can be removed in case it was positioned on the table 10 at a position at which a patient needs to be subsequently positioned. Consequently, the position of the patient 11 can be correlated with the displayed diagnostic image 13, as shown in Fig. 5B and no further reference means need to be used during the subsequent procedure.

The reference point indicator 32 has a base 40 for positioning the indicator 32 on a surface, such as the table, a telescopic arm 41 carrying a set of markers 42 (such as matrix codes) and a head 43 mounted at a fixed position relative to the markers 42. The markers 42 are recognizable by the point position recognition module 30, which can then determine the position of the head 43 based on the detected position of the markers 42. The length of the telescopic arm 41 can be adjusted, for example between 5 and 50 cm, so as to allow positioning the head 43 at a desired location of the gantry reference point 31.

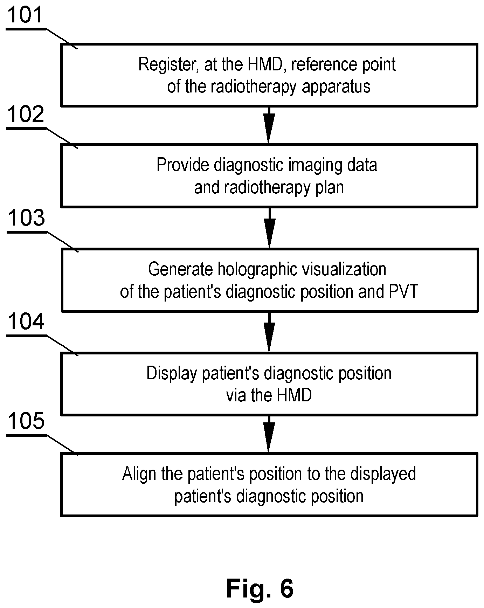

To summarize, an embodiment of a patient positioning procedure is shown in Fig. 6. First, in step 101, a position of a reference point of the radiotherapy apparatus is registered by a reference point position recognition module 30 and provided to the HMD 15. For example, the reference point may be registered using the reference point indicator 32 positioned at a crossing point of laser rays R1, R2 emitted by the laser pointers 51, 52. Then, in step 102, diagnostic imaging data 23 (with a defined position of reference point 22) and radiotherapy plan 21 are sent from a planning tool 25 to the visualization engine 26 in the computer workstation. In step 103, the visualization engine 26 generates a holographic visualization of the patient's diagnostic position and (optionally) the PVT, that is sent to the HMD 15 worn by the medical practitioner 14, and optionally also a secondary image to be displayed via the 3D visualization module 28. In step 104, the holographic visualization is displayed as the patient's diagnostic imaging position 13 via the HMD 15, wherein the reference point of the diagnostic image is aligned with the gantry reference point. At this stage, in step 105, the medical practitioner may request the patient to lay on the table 10 in a position 11 corresponding to the diagnostic image position 13 and correct the patient's position, if necessary, i.e., by shifting the patient's location on the table or correcting the patient's pose. Step 105 can be computer-assisted by the position evaluation module 29. Once the patient is positioned in alignment with the diagnostic image position, radiotherapy can be initiated, in particular by delivering radiation to the PTV

While the invention has been described with respect to a limited number of embodiments, it will be appreciated that many variations, modifications, and other applications of the invention may be made. Therefore, the claimed invention, as recited in the claims that follow, is not limited to the embodiments described herein.

## Claims

1. A method for positioning a patient (11) on a table (10) in preparation for radiation therapy to be delivered by a radiotherapy apparatus, the method comprising:
- registering (101), at a head-mounted display, HMD (15) worn by a medical practitioner (14), a position of a reference point (31) of a radiotherapy apparatus;
- providing (102) diagnostic imaging data (23) of the patient, including the patient's diagnostic position, wherein the diagnostic imaging data (23) is defined with reference to a diagnostic reference point (22);
- generating (103), by the visualization engine, a holographic visualization of the patient's diagnostic imaging position based on the diagnostic imaging data; and
- displaying (104) the holographic visualization of the patient's diagnostic imaging position (13) over the patient (11) using augmented reality or mixed reality technology of the HMD (15) to allow the medical practitioner (14) to align the current position of the patient with the patient's diagnostic imaging position (13).

2. The method according to claim 1, further comprising providing (102), along with the diagnostic imaging data (23), a radiotherapy plan (21) that includes the location of the diagnostic planning target volume (13P) and displaying the diagnostic planning target volume (13P) via the HMD (15).

3. The method according to any of previous claims, comprising indicating, during the alignment (105) of the patient's position, a level of compliance between the patient's current position (11) and the holographic visualization of the diagnostic position (13).

4. The method according to any of previous claims, further comprising generating (104) a secondary image of the patient's diagnostic imaging position to be displayed outside the radiotherapy room.

5. A system for positioning a patient (11) on a table (10) in preparation for radiation therapy to be delivered by a radiotherapy apparatus, the system comprising:
- a head-mounted display, HMD (15) to be worn by a medical practitioner (14); and
- a computer workstation (24) communicatively coupled with the HMD (15) and includes a processor and memory storing data and software, wherein the software includes a planning tool (25) and a visualization engine (26) configured to perform the steps of the method according to any of previous claims.

6. The system according to claim 5, further comprising a reference point indicator (32) configured to establish a reference point corresponding to a gantry reference point (31) of a radiotherapy apparatus, comprising a base (40) carrying a telescopic arm (41) on which there are mounted markers (42) and a head (43).

7. The system according to claim 6, further comprising laser pointers (51, 52) for emitting laser rays (R1, R2) that intersect at the gantry reference point (31).

8. The system according to claim 7, further comprising a reference point position recognition module (30) configured to register the position of the head (43) of the reference point indicator (32).

9. The system according to any of claims 5-8, comprising a wireless communication interface (27) for transmitting holographic data from the workstation (24) to the HMD (15).

10. The system according to any of claims 5-9, further comprising a position evaluation module (29) configured to determine compliance between the patient's current position (11) and the holographic visualization of the diagnostic imaging position (13) and to provide feedback corresponding to the level of compliance.

11. The system according to claim 10, wherein the feedback provided by the position evaluation module (30) includes at least one of an audio signal or a visual modification of the holographic image (13).

12. The system according to any of claims 5-10, further comprising a 3D visualization module (28) located outside the radiotherapy room for displaying a secondary image of the diagnostic imaging position (13) and the patient (11).
